# EUROPEAN PATENT APPLICATION

(11) **EP 4 454 680 A1**
(43) Date of publication of application: **30.10.2024**
(21) Application number: 22911532.4
(22) Date of filing: 30.09.2022
(51) Int. Cl.: A61M 5/145, A61M 5/142

(54) **ELECTROOSMOTIC PUMP**

(30) Priority: 24.12.2021 KR 20210187463
(71) Applicant: Eoflow Co., Ltd., Seongnam-si, Gyeonggi-do 13605 (KR)
(72) Inventor: LEE, Do Kyung, Hwaseong-si Gyeonggi-do 18466 (KR); JEON, Joon Sung, Yongin-si Gyeonggi-do 16902 (KR); YUN, Kwang Sik, Hwaseong-si Gyeonggi-do 18466 (KR)
(74) Representative: Meissner Bolte Partnerschaft mbB
(86) International application number: PCT/KR2022/014727
(87) International publication number: WO 2023/120893

(57) **Abstract**

An electroosmotic pump according to an embodiment of the present disclosure may include: a housing that provides an inner space for containing a fluid therein and has a shaft hole connecting the inner space to an outer space thereof; and a shaft assembly that covers the shaft hole from an outside of the housing, is connected to the housing, and is capable of changing a shape in a longitudinal direction by a flow of the fluid passing through the shaft hole.

## Description

### Technical Field

The present disclosure relates to an electroosmotic pump, and more particularly, to an electroosmotic pump using a fluid.

### Background Art

Generally, liquid medicine injection devices, such as insulin injection devices, are used to inject liquid medicine into the body of a patient. Such liquid medicine injection devices are used by professional medical staffs, such as medical doctors and nurses. However, in most cases, liquid medicine injection devices are used by the general public, such as patients themselves or guardians thereof.

In this regard, patch-type liquid medicine injection devices that are easily used while being attached to the human body for a certain period of time are under development. Such liquid medicine injection devices may be used while being attached to the body of the patient, such as the abdomen or waist of the patient, in the form of a patch for a certain period of time.

A liquid medicine injection device may include a driving member, such as an electroosmotic pump, so as to actively inject liquid medicine. The electroosmotic pump is a pump that utilizes a fluid movement phenomenon occurring when a voltage is applied across a capillary tube or a porous separation film.

The electroosmotic pump includes a piston capable of performing a linear reciprocating motion according to the movement of fluid in a portion formed in a cylindrical structure therein, and the piston may be coupled to a shaft to cause the shaft to perform a linear reciprocating motion. One end of the shaft may be coupled to the piston within the pump, and the other end of the shaft may be exposed to the outside of the pump and coupled to a moving member provided outside the pump. The moving member may perform a linear reciprocating motion in conjunction with a motion of the piston and shaft and may transmit power to a liquid medicine discharge mechanism.

When the piston reciprocates within the pump, the lateral force that crosses the direction of movement of the shaft may act on the shaft. The lateral force may be generated by several factors, such as vibration that occurs when the pump operates or shock that is applied from the outside. At this time, the alignment state of the piston that performs a linear reciprocating motion in a cylinder part may be disturbed, thus causing a problem that a fluid inside the pump leaks through between the cylinder part and the piston.

### Disclosure

### Technical Problem

Various types of driving members, such as motors or pumps, may be used in a mechanism for driving a medicine injection device such as an insulin injection device. The present disclosure relates to a driving member capable of performing fine pumping by using a fluid and is intended to prevent a fluid inside a pump from leaking to the outside through a piston during pumping. However, this is only an example and the scope of the present disclosure is not limited thereby.

### Technical Solution

As a means for achieving the technical objects described above, an electroosmotic pump according to an embodiment of the present disclosure may include: a housing that provides an inner space for containing a fluid therein and has a shaft hole connecting the inner space to an outer space thereof; and a shaft assembly that covers the shaft hole from an outside of the housing, is connected to the housing, and is capable of changing a shape in a longitudinal direction by a flow of the fluid passing through the shaft hole.

In an embodiment, the shaft assembly may include: an expansion/contraction member that forms an expansion/contraction space communicating with the shaft hole; and a rod member that is coupled to the expansion/contraction member and is formed to extend while sharing a longitudinal central axis with the expansion/contraction member.

In an embodiment, the expansion/contraction member may include: a body part that has an expansion/contraction space provided therein with an opening facing the shaft hole and is formed in a shape of a corrugated pipe; a flange part extending radially from one end of the body part having the opening formed therein; and a head part formed on another end opposite to the one end of the body part from which the flange part extends, and the flange part may be joined to the housing.

In an embodiment, the expansion/contraction member may be formed of a rubber material.

In an embodiment, the electroosmotic pump may further include a membrane that divides the inner space into a first space and a second space, wherein the flow of the fluid passing through the shaft hole may depend on a flow of the fluid passing through the membrane.

Aspects, features, and advantages other than those described above will become better understood through the accompanying drawings, the claims, and the detailed description.

### Advantageous Effects

An electroosmotic pump according to an embodiment of the present disclosure may stably secure airtightness of a pump. Accordingly, a fluid inside the pump may be prevented from leaking to the outside during a pump operation, thus preventing the performance deterioration of the pump.

In addition, a coating process required between an outer circumferential surface of a piston and an inner circumferential surface of a cylinder may be omitted. Silicone oil may be used in the coating process. By omitting the coating process, a problem caused by silicone oil (for example, bubble generation) may be prevented.

Since the piston and the coating process may be omitted when manufacturing the pump, the time and cost required to manufacture the pump may be saved.

However, these effects are only examples and the scope of the present disclosure is not limited by such effects.

### Description of Drawings

FIG. 1 is a perspective view illustrating an electroosmotic pump according to an embodiment of the present disclosure.
FIG. 2 is a diagram illustrating a shaft assembly coupled to a housing according to an embodiment of the present disclosure.
FIGS. 3A and 3B are diagrams illustrating that the shape of the shaft assembly is deformed by the flow of fluid contained in the pump.
FIG. 4 is a diagram illustrating the inside of the pump of FIG. 1.
FIGS. 5A and 5B are schematic diagrams illustrating reactions in a first electrode body and a second electrode body with respect to a membrane, according to an embodiment of the present disclosure.
FIGS. 6A and 6B are diagrams for describing the operation of the pump according to an embodiment of the present disclosure.
FIG. 7 is a graph showing a ratio of a volume of a second sub-space to a volume of a first space according to a reciprocating motion of a shaft.

### Mode for Disclosure

As the present description allows for various changes and numerous embodiments, certain embodiments will be illustrated in the drawings and described in detail in the written description. Effects and features of the present disclosure, and methods of achieving them will be clarified with reference to embodiments described below in detail with reference to the drawings. However, the present disclosure is not limited to the following embodiments and may be embodied in various forms.

Hereinafter, embodiments will be described in detail with reference to the accompanying drawings. When describing embodiments with reference to the accompanying drawings, the same or corresponding elements are denoted by the same reference numerals, and redundant descriptions thereof are omitted.

In the following embodiments, the terms "first," "second," etc. are not used in a restrictive sense and are used to distinguish one element from another.

In the following embodiments, the singular forms are intended to include the plural forms as well unless the context clearly indicates otherwise.

In the following embodiments, the terms "comprise" or "include" specify the presence of stated features or elements, but do not preclude the presence or addition of one or more other features or elements.

Sizes of elements in the drawings may be exaggerated or reduced for convenience of explanation. For example, since sizes and thicknesses of elements in the drawings are arbitrarily illustrated for convenience of explanation, the following embodiments are not limited thereto.

In the following embodiments, regions or elements are referred to as being connected to each other, the regions or the elements may be directly connected to each other or indirectly connected to each other with intervening regions or elements therebetween.

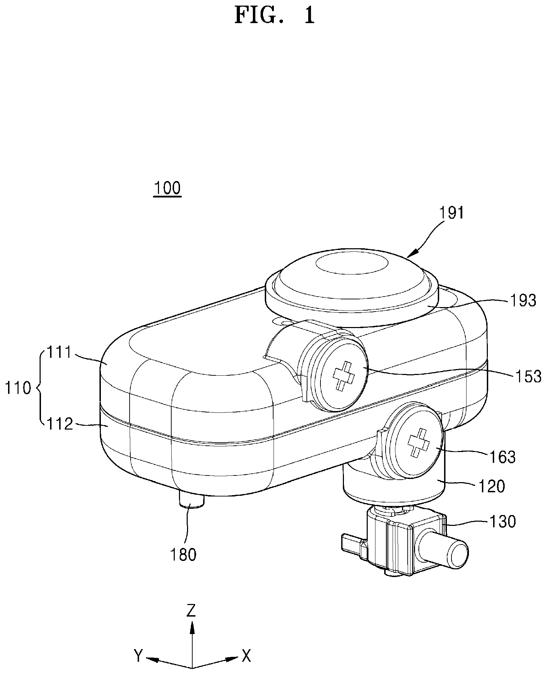

FIG. 1 is a perspective view of an electroosmotic pump according to an embodiment of the present disclosure.

Referring to FIG. 1, an electroosmotic pump (hereinafter referred to as a 'pump') 100 may include a housing 110 and a shaft assembly 120.

The housing 110 may form the outer appearance of the pump 100 and may provide an inner space in which various components provided in the pump 100, including a fluid used to operate the pump 100, may be accommodated. The housing 110 may include a first housing 111 and a second housing 112 and may be configured such that the first housing 111 and the second housing 112 are assembled with each other.

The housing 110 may include a first terminal 153 and a second terminal 163 that may be electrically connected to an electrode body. The housing 110 may have an injection port 180 through which the fluid may be injected into the housing 110. The housing 110 may be provided with a deformation part 191 to cope with a pressure change generated by the flow of fluid within the housing 110. The first terminal 153, the second terminal 163, the injection port 180, and the deformation part 191 will be described again in detail.

The pump 100 may be operated by an electrical signal applied to the pump 100, and the shape of the shaft assembly 120 may be deformed in the Z direction by the operation of the pump 100.

The shaft assembly 120 may be arranged outside the housing 110, such that one side thereof is installed in the housing 110 and the other side thereof is coupled to a moving member 130. The moving member 130 is connected to another driving mechanism to transmit power. As the shape of the shaft assembly 120 is deformed between the pump and the other driving mechanism, the moving member 130 may be configured to perform a linear reciprocating motion and transmit power to the other driving mechanism.

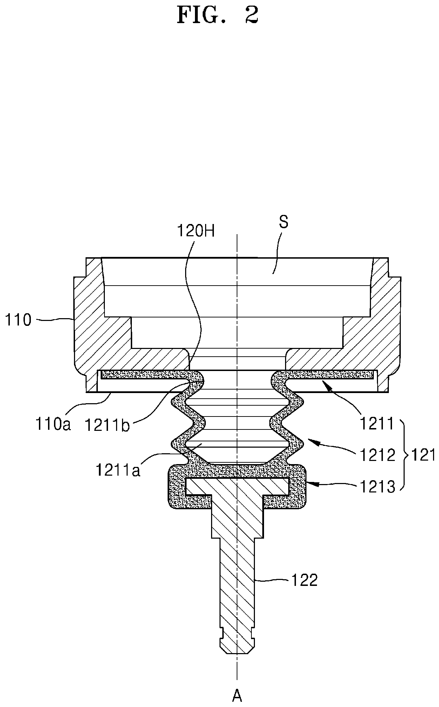

FIG. 2 is a diagram illustrating the coupled state of the housing 110 and the shaft assembly in FIG. 1.

A shaft hole 120H may be provided at one side of the housing 110. The shaft hole 120H may be provided in the form of a through-hole passing through the inner circumferential surface and the outer circumferential surface of the housing 110. The inside and the outside of the housing 110 may communicate with each other through the shaft hole 120H. The fluid contained in the inner space S of the housing 110 may flow from the inside to the outside of the housing 110 and from the outside to the inside of the housing 110 through the shaft hole 120H.

The shaft assembly may be coupled to the outside of the housing 110. The shaft assembly may be coupled to a portion where the shaft hole 120H is formed and may cover the shaft hole 120H in the outside of the housing 110. The shaft assembly is tightly fixed to the housing 110 so as to prevent the fluid from leaking from the joint portion of the housing 110 and the shaft assembly, thereby ensuring airtightness at the joint portion.

As the fluid contained in the housing 110 flows through the shaft hole 120H, the shape of the shaft assembly may be deformed in the longitudinal direction A.

The shaft assembly may include an expansion/contraction member 121 and a rod member 122.

The expansion/contraction member 121 may be formed of an elastic hydrocarbon polymer (rubber). One side of the expansion/contraction member 121 may be fixed to the housing 110 and the other side of the expansion/contraction member 121 may be coupled to the rod member 122. One side of the rod member 122 may be coupled to the expansion/contraction member 121 and may extend in the longitudinal direction A. The rod member 122 may share a central axis with the expansion/contraction member 121. One side of the expansion/contraction member 121 may be expanded and contracted while being fixed to the housing 110, and thus, the rod member 122 coupled to the other side of the expansion/contraction member 121 is allowed to linearly reciprocate in the longitudinal direction A.

The expansion/contraction member 121 may include a body part 1211, a flange part 1212 provided at one end of the body part 1211, and a head part 1213 provided at the other end of the body part 1211.

The body part 1211 may be formed in the form of a corrugated pipe so as to have elasticity. The body part 1211 may be expanded and contracted while the shape of the body part 1211 is changed by force applied thereto in the longitudinal direction A.

The body part 1211 may provide an expansion/contraction space 1211a. The expansion/contraction space 1211a is a space formed inside the body part 1211. The expansion/contraction space 1211a may have an opening 1211b with one side open and may have a size (volume) that varies depending on the expansion and contraction of the body part 1211.

When the expansion/contraction member 121 is coupled to the housing 110, the opening 1211b of the expansion/contraction space 1211a may face the shaft hole 120H. Accordingly, the expansion/contraction space 1211a may communicate with the inner space S provided inside the housing 110.

The flange part 1212 may be formed to facilitate coupling with the housing 110. For example, the flange part 1212 may be formed to extend radially from the open end of the body part 1211 and may provide a surface that may be joined to the housing 110. A seating groove 110a may be formed in the housing 110 around the shaft hole 120H to facilitate joining with the flange part 1212.

The head part 1213 may be provided on the other side of the body part 1211 and coupled to the rod member 122. The head part 1213 may have a certain thickness to enable coupling with the rod member 122. The rod member 122 coupled to the head part 1213 may perform a linearly reciprocating motion in the longitudinal direction A as the body part 1211 is expanded and contracted.

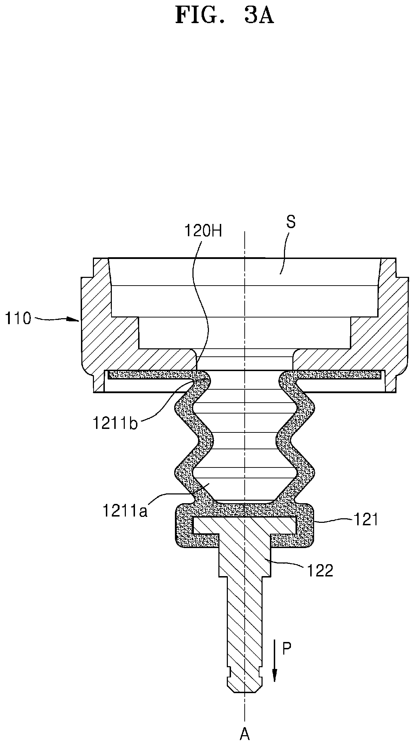

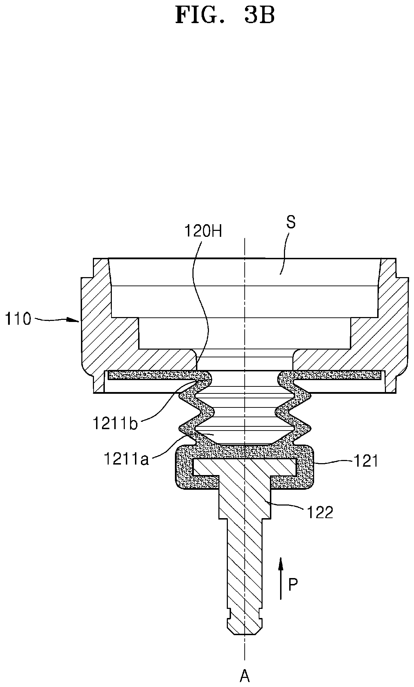

FIGS. 3A and 3B are diagrams illustrating that the shape of the shaft assembly is deformed by the flow of fluid contained in the pump.

When the pump operates, the pressure change and the resulting fluid flow may occur in the inner space S of the housing 110.

Referring to FIG. 3A, the fluid in the inner space S may be introduced into the expansion/contraction space 1211a through the shaft hole 120H and the opening 1211b, depending on the pressure change in the inner space S. Due to the introduction of the fluid, the size of the expansion/contraction space 1211a may increase and the expansion/contraction member 121 may be expanded in the longitudinal direction A. At this time, the rod member 122 fixedly coupled to the expansion/contraction member 121 may linearly move in a direction away from the housing 110 in an extension direction P1 of the expansion/contraction member 121.

Referring to FIG. 3B, the fluid contained in the expansion/contraction space 1211a may be introduced into the inner space S of the housing 110 through the opening 1211b and the shaft hole 120H, depending on the pressure change in the inner space S. Due to the introduction of the fluid, the size of the expansion/contraction space 1211a may decrease and the expansion/contraction member 121 may be contracted in the longitudinal direction A. At this time, the rod member 122 fixedly coupled to the expansion/contraction member 121 may linearly move toward the housing 110 in a contraction direction P2 of the expansion/contraction member 121.

While the pump operates, the fluid used to change the shape of the shaft assembly may flow only in the space S (1211a) provided by the housing 110 and the shaft assembly, that is, may flow only in the pump. Since the housing 110 and the shaft assembly are operated while being coupled to each other in a state of ensuring airtightness, the fluid does not leak even when vibration or external impact occurs due to the operation of the pump. Accordingly, the performance of the pump may be kept constant.

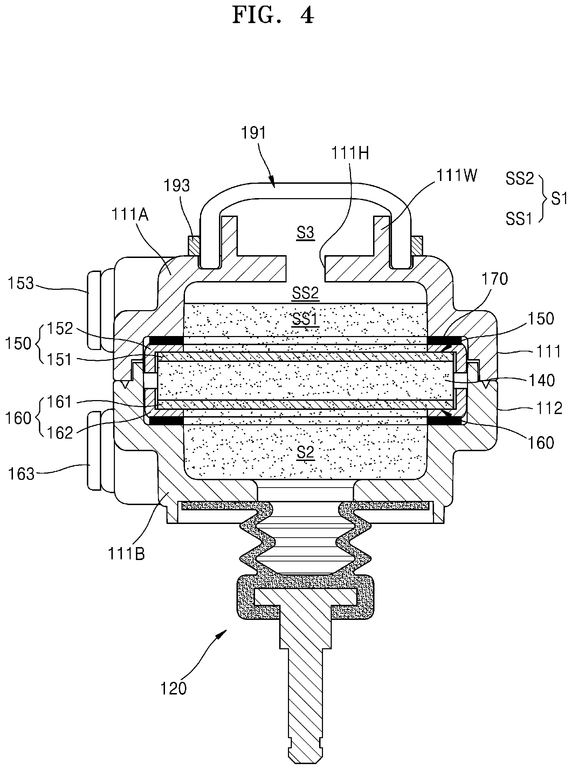

FIG. 4 is a diagram illustrating the inside of the pump 100 of FIG. 1.

The membrane 140 may be arranged in the inner space of the housing 110. The first housing 111 and the second housing 112 may be coupled to each other with the membrane 140 therebetween. The inner space may include a first space S1 and a second space S2 respectively located on both sides of the membrane 140.

In the drawing, a space far from the shaft assembly 120 with respect to the membrane 140 is represented as the first space S1, and a space adjacent to the shaft assembly 120 with respect to the membrane 140 is represented as the second space S2.

The membrane 140 may have a porous structure that allows the fluid and ions to move. The membrane 141 may be, for example, a frit-type membrane manufactured by sintering spherical silica with heat. For example, the spherical silica used to form the membrane may have a diameter of about 20 nm to about 500 nm, specifically about 30 nm to about 300 nm, and more specifically, about 40 nm to about 200 nm. When the diameter of the spherical silica satisfies the range described above, pressure due to the first fluid passing through the membrane 140, that is, pressure sufficient to deform the shape of the shaft assembly 120, may be generated.

In the embodiment described above, it has been described that the membrane 140 includes the spherical silica, but the membrane 140 is not limited thereto.

As another embodiment, the type of the membrane 140 is not limited as long as the membrane 140 is formed of a material capable of causing an electrokinetic phenomenon due to zetapotential, such as porous silica or porous alumina.

The membrane 140 may have a thickness of about 20 µm to about 10 mm, specifically about 300 µm to about 5 mm, and more specifically, about 1,000 µm to about 4 mm.

A first electrode body 150 and a second electrode body 160 may be respectively arranged on both sides of the membrane 140. The first electrode body 150 may include a first porous plate 151 and a first strip 152 arranged on a first side of the membrane 140. The second electrode body 160 may include a second porous plate 161 and a second strip 162 arranged on a second side of the membrane 140.

The first porous plate 151 and the second porous plate 161 may be respectively arranged to be in contact with main surfaces on both sides of the membrane 140. The first porous plate 151 and the second porous plate 161 may effectively move the fluid and ions through the porous structures thereof.

The first porous plate 151 and the second porous plate 161 may have a structure in which an electrochemical reactant is formed in a porous base layer. For example, the electrochemical reactant may be formed by electrodeposition or coating on the porous base layer through methods such as electroless plating, vacuum deposition, coating, and sol-gel process.

The porous base layer may be an insulator. For example, the porous base layer may include one or more selected from non-conductive ceramic, non-conductive polymer resin, non-conductive glass, and any combination thereof.

The non-conductive ceramic may include, for example, one or more selected from the group consisting of rock wool, gypsum, ceramic, cement, and any combination thereof, and specifically, may include one or more selected from the group consisting of rock wool, gypsum, and any combination thereof, but the present disclosure is not limited thereto.

The non-conductive polymer resin may include, for example, one or more selected from the group consisting of: synthetic fiber selected from the group consisting of polypropylene, polyethylene terephthalate, polyacrylonitrile, and any combination thereof; natural fiber selected from the group consisting of wool, cotton, and any combination thereof; sponge; a porous material derived from living organisms, for example, the bones of living organisms; and any combination thereof, but the present disclosure is not limited thereto.

The non-conductive glass may include one or more selected from the group consisting of glass wool, glass frit, porous glass, and any combination thereof, but the present disclosure is not limited thereto.

The porous base layer may have a pore size of about 0.1 µm to about 500 µm, specifically about 5 µm to about 300 µm, and more specifically, about 10 µm to about 200 µm.

When the pore size of the porous support satisfies the range described above, the fluid and ions may be effectively moved, thereby improving the stability, lifespan characteristics, and efficiency of the pump 100.

The electrochemical reactant may include a material that may form a pair of reactions in which an oxidizing electrode and a reducing electrode exchange cations, for example, hydrogen ions during an electrode reaction of the first electrode body 150 and the second electrode body 160, and at the same time, may form a reversible electrochemical reaction.

The electrochemical reactant may include, for example, silver/silver oxide, silver/silver chloride, MnO(OH), polyaniline, polypyrrole, polythiophene, polythionine, quinone-based polymer, and any combination thereof.

The first strip 152 and the second strip 162 may be respectively arranged at the edges of the first porous plate 151 and the second porous plate 161, and may be connected to a first terminal 153 and a second terminal 163 arranged outside the housing 110. The first strip 152 and the second strip 162 may each include a conductive material, such as silver or copper.

The fluid provided in the inner space of the housing 110 may include a first fluid and a second fluid having different phases. The first fluid may include a liquid such as water and the second fluid may include a gas such as air.

The first fluid existing in the inner space may not completely fill the inner space. That is, the volume of the inner space is greater than the volume of the first fluid existing in the inner space. The second fluid may exist in a portion of the inner space where the first fluid does not exist.

A sealing material 170 may be arranged on either side of the structure of the membrane 140, the first electrode body 150, and the second electrode body 160. The sealing material 170 may be formed in a ring shape with the area corresponding to the edge of the structure described above.

The fluid described above, for example, the first fluid moves from the first space S1 to the second space S2 or vice versa in the thickness direction of the membrane 140 so as to pass through the membrane 140. At this time, the sealing material 170 may block a gap between the inner surface of the housing 110 and the above-described structure, thereby preventing the fluid from moving into the gap.

The fluid may flow into the inner space through the injection port 180, as illustrated in FIG. 1. [113] As an embodiment, after the entire inner space is filled with the first fluid through the injection port 180 on one side, a part of the first fluid is taken out to the outside through the injection port 180, and then, the injection port 180 is closed. Accordingly, the first fluid and the second fluid may exist in the inner space of the housing 110.

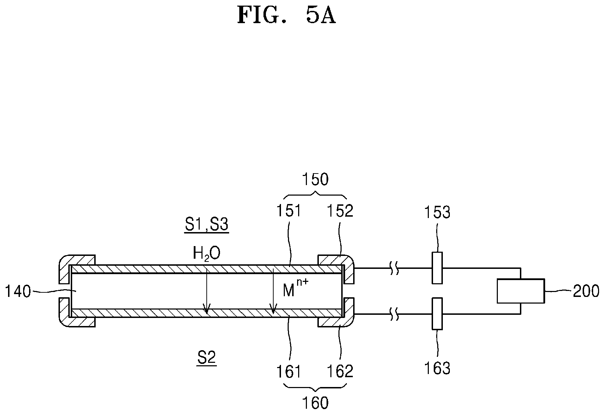

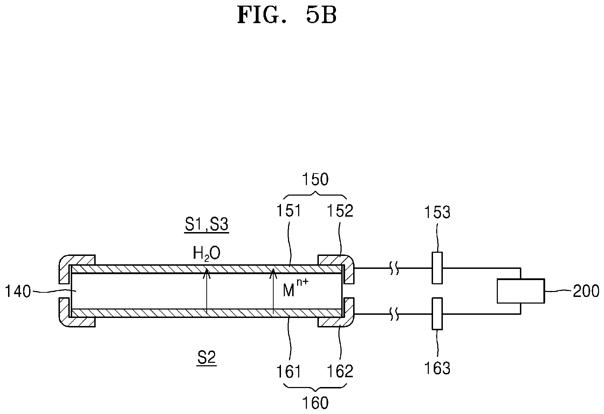

FIGS. 5A and 5B are schematic diagrams illustrating the reaction in the first electrode body 150 and the second electrode body 160 with respect to the membrane.

Referring to FIGS. 5A and 5B, the first electrode body 150 and the second electrode body 160 may be electrically connected to a power supply part 200 through the first terminal 153 and the second terminal 163, respectively. By alternately changing the polarity of the voltage supplied by the power supply part 200, the direction of movement of the liquid such as water may be changed.

A case where silver/silver oxide is used as the electrochemical reactant and the first fluid is a solution including water is described.

As illustrated in FIG. 5A, when the first electrode body 150 is an oxidizing electrode and the second electrode body 160 is a reducing electrode, the reaction of Ag(s)+H2O→Ag2O(s)+2H++2e- occurs in the first electrode body 150, and the reaction of Ag2O(s)+2H++2e-→Ag(s)+H2O occurs in the second electrode body 160.

Cations (Mⁿ⁺, for example, hydrogen ions) generated according to the oxidation reaction in the first electrode body 150 pass through the membrane 140 and move toward the second electrode body 160 by the voltage difference. At this time, a certain pressure may be generated while water (H2O) is moving together with cations.

Thereafter, when the polarity of the voltage supplied by the power supply part 200 is reversed as illustrated in FIG. 5B, the electrochemical reactant consumed when used as the oxidizing electrode is recovered when used as the reducing electrode. Similarly, the reducing electrode is also recovered. Accordingly, the first electrode body 150 and the second electrode body 160 may continuously react according to the voltage supply of the power supply part 200. Unlike in FIG. 5A, when the polarity of the voltage supplied to the first electrode body 150 and the second electrode body 160 is changed, cations (Mⁿ⁺, for example, hydrogen ions) and water (H2O) move from the second space S2 back to the first space S1, as illustrated in FIG. 5B.

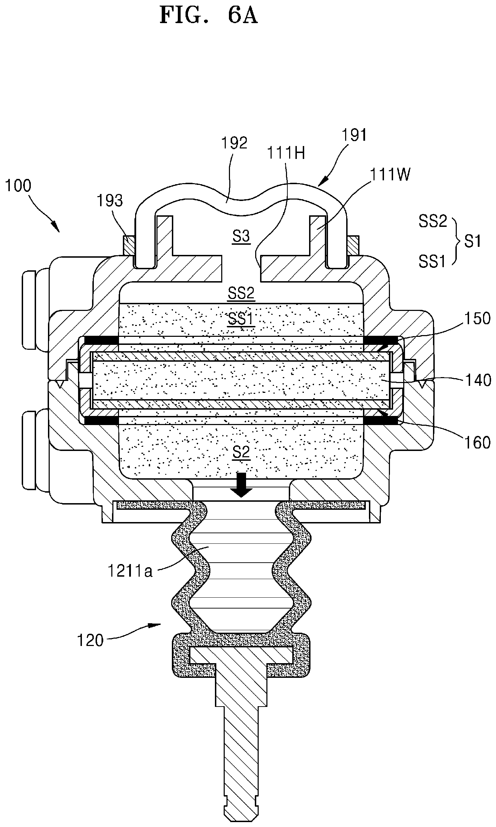

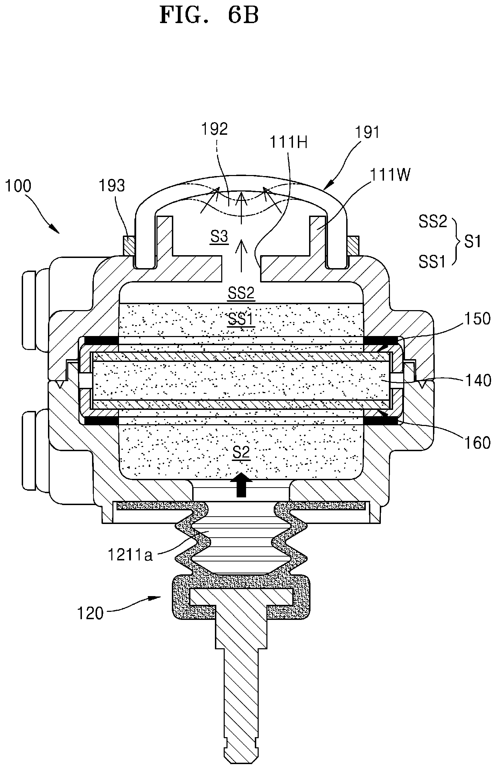

FIGS. 6A and 6B are diagrams for describing the operation of the pump 100 according to an embodiment of the present disclosure. FIG. 6A illustrates a state in which the fluid on the housing 110 side moves toward the shaft assembly 120 by the operation of the pump 100, and FIG. 6B illustrates a state in which the fluid on the shaft assembly 120 side moves toward the housing 110 by the operation of the pump 100.

Referring to FIGS. 6A and 6B, the first fluid such as water exists in the inner space of the housing 110, and the volume of the first fluid existing in the inner space is less than the volume of the inner space. The second fluid including a gas such as air may exist in a portion of the inner space where the first fluid does not exist, specifically in the first space S1 of the housing 110 and the third space S3 that is the space of the deformation part 191.

For example, the first fluid exists in each of the first space S1 and the second space S2. The first fluid and the second fluid coexist in the first space S1, and the volume of the first fluid existing in the first space S1 may be less than the volume of the first space S1.

The first fluid also exists in the second space S2. However, unlike the first space S1, the second fluid does not exist in the second space S2. For convenience of explanation, a portion of the first space S1 where the first fluid, which is a liquid, exists is referred to as a first sub-space SS1, and a portion of the first space S1 where the second fluid, which is a gas, exists is referred to as a second sub-space SS2. The first sub-space SS1 and the second sub-space SS2 may form the first space S1. For example, the remaining portion of the first space S1 other than the first sub-space SS1 may be the second sub-space SS2.

The second fluid may exist in the first space S1, specifically the second sub-space SS2, and the third space S3 that is the inner space of the deformation part 191 communicating therewith.

When the power supply part 200 supplies voltage to the first electrode body 150 and the second electrode body 160 as described with reference to FIG. 5A, the reaction described with reference to FIG. 5A occurs. As illustrated in FIG. 6A, cations (e.g., hydrogen ions) may move in a first direction (-Z direction in FIG. 6B) from the first space S1 toward the second space S2.

At this time, pressure is generated as the first fluid (e.g., H2O) in the first space S1 passes through the membrane 140 together with cations and moves in the first direction (the -Z direction in FIG. 6B). Due to the pressure, the first fluid may move from the housing 110 side to the expansion/contraction space 1211a provided in the shaft assembly 120 side, specifically the expansion/contraction member.

Referring to FIGS. 6A and 6B, the deformation part 191 according to an embodiment of the present disclosure may communicate with one side (the upper side in FIG. 6A) of the housing 110 where the first space S1 is formed, and may communicate with the first space S1 and the third space S3, which is the inner space of the deformation part, through a hole 111H formed in one surface (the upper surface in FIG. 6A) of the housing 110 facing the deformation part 191.

An elastic part 192 that is elastically deformable may be formed on one side of the deformation part 191 according to an embodiment of the present disclosure. The elastic part 192 may be formed in the central portion of the deformation part 191 and may be formed to be concave or convex with respect to the outward direction (the upward direction in FIG. 6A) of the deformation part 191.

As the shape of the elastic part 192 is deformed, the volume of the third space S3 may increase or decrease.

The shape of the elastic part 192 may be deformed depending on the inner pressure of the inner space of the deformation part 191, specifically the third space S3. Referring to FIG. 6A, negative pressure may be formed in the third space S3.

The elastic part 192 may have an elastic restoring force in a direction in which the elastic part 192 is formed to be convex with respect to the outward direction (the upper direction in FIG. 6A) of the deformation part 191. This provides an effect in which, when the first fluid moves from the second space S2 to the first space S1 together with cations, as illustrated in FIG. 6B, the space in which the second fluid, such as air, is contained is secured as much as the third space S3, and the force required to reduce the expansion/contraction member may be relatively reduced, and the reduction of the expansion/contraction member is facilitated.

As the first fluid (e.g., H2O) in the first space S1 moves to the second space S2, the volume ratio of the first sub-space SS1 to the volume of the first space S1 decreases, and the proportion occupied by the second sub-space SS2 in the first space S1 increases.

Meanwhile, when the power supply part 200 changes the polarity of the voltage and supplies the voltage to the first electrode body 150 and the second electrode body 160, as described with reference to FIG. 5B, cations (e.g., hydrogen ions) and the first fluid (e.g., water) move in a second direction (+Z direction in FIG. 6) from the second space S2 toward the first space S1. As illustrated in FIG. 6B, as the fluid in the expansion/contraction space 1211a moves toward the housing 110, the shaft assembly 120 may move back to the original position thereof.

While the shaft assembly 120 moves in the second direction (the +Z direction in FIG. 6B), the first fluid moves together with cations in the second direction, and the shape of the deformation part 191, specifically the elastic part 192, may be deformed.

Specifically, as the elastic part 192 is deformed from a concave shape to a convex shape with respect to the outward direction of the deformation part 191, there is an effect in which the volume of the third space S3 increases and the reduction of the expansion/contraction member is facilitated.

In addition, when compression occurs in the first space S1 and the third space S3, the deformation part 191, specifically the elastic part 192, has an elastic restoring force in a direction in which the elastic part 192 is formed to be convex. Accordingly, there is an effect of further facilitating the reduction of the expansion/contraction member.

In addition, when the reaction in FIGS. 5A and 5B occurs and gas is generated, the gas may be contained in the third space S3. Accordingly, there is an effect of performing a buffer function.

When the power supply part 200 alternately changes the polarity of the voltage applied to the first electrode body 150 and the second electrode body 160, the expansion/contraction member repeats expansion and contraction, and thus, the rod member coupled to the expansion/contraction member may perform a linearly reciprocating motion in the first direction and the second direction.

The expansion and contraction of the expansion/contraction member and the reciprocating motion of the rod member may be described as the change according to the volume ratio of a portion of the first space S1 where the second fluid exists, that is, the second sub-space SS2.

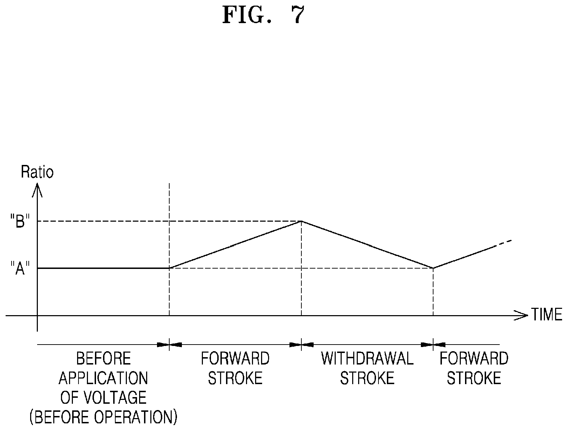

FIG. 7 is a graph showing the ratio of the volume VSS2 of the second sub-space to the volume VS1 of the first space according to the shape deformation of the shaft assembly.

When the ratio (=VSS2/VS1) of the volume of the second sub-space SS2 to the volume of the first space S1 in a state before the power supply part 200 applies voltage to the first electrode body 150 and the second electrode body 160, that is, in a state before the pump 100 operates is "A," the ratio during the forward stroke of the shaft assembly 120, that is, when the shape is deformed in the first direction, increases to "B" (A<B, where A is greater than 0 and B is less than 1).

In the stroke where the shaft assembly 120, which had advanced, withdraws in the second direction, the above-described ratio decreases from B to A, but the ratio does not become less than A.

The pump 100 according to an embodiment of the present disclosure may be provided by assembling the respective components in a state in which the expansion/contraction member is relatively contracted. As another embodiment, the pump 100 may be provided by assembling the respective components in a state in which the expansion/contraction member is relatively expanded.

When the expansion/contraction member is expanded or contracted, the second fluid (e.g., air) existing in the second sub-space SS2 and the third space S3 may be slightly expanded or compressed. A certain force may be stored in the second fluid depending on the expansion or compression of the second fluid, and this force may act on the expansion and contraction of the expansion/contraction member. Accurate control of the expansion/contraction stroke of the expansion/contraction member may affect the amount of medicine injected in the medicine injection device in which the pump 100 is used.

Therefore, the expansion/contraction stroke of the expansion/contraction member may be designed, for example, taking into account the aforementioned force.

Referring to FIGS. 6A to 6B, the elastic part 192 that is elastically deformable may be formed on one side (the upper side in FIGS. 6A and 6B) of the deformation part 191 according to an embodiment of the present disclosure. As the elastic part 192 is elastically deformed, the volume of the third space S3 may be deformed.

When the reaction of FIG. 5A occurs, negative pressure is formed in the third space S3 and, as illustrated in FIG. 6A, the shape of the elastic part 192 may be formed to be concave with respect to the outward direction of the deformation part 191.

When the reaction of FIG. 5B occurs, the pressure increases due to the pressure of the first fluid, etc. and, as illustrated in FIG. 6B, the elastic part 192 has an elastic restoring force in a direction of being formed to be convex with respect to the outward direction of the deformation part 191. Accordingly, the shape of the elastic part 192 may be deformed in a direction in which the volume of the third space S3 increases. By removing the force stored in the second fluid, there is an effect of facilitating the reduction of the expansion/contraction member and accurately controlling the expansion and contraction of the expansion/contraction member, preferably, the movement of the rod member connected to the expansion/contraction member.

Referring to FIGS. 1, 4, 6A, to 6B, a fixing member 193 according to an embodiment of the present disclosure is coupled to each of the housing 110 and the deformation part 191, and the position of the deformation part 191 may be fixed on the housing 110.

In addition, there is an effect of blocking the second fluid including air from leaking out of the third space S3 that is the inner space of the deformation part 191.

The fixing member 193 may be in close contact with the outer circumferential surface of the deformation part 191 and may be installed in the housing 110.

A sealing wall 111W, which protrudes outward and is in close contact with the inner circumferential surface of the deformation part 191, may be formed to protrude on one surface of the housing 110 facing the deformation part 191.

Due to the sealing wall 111W, the inner circumferential surface of the deformation part 191 is in close contact with the sealing wall 111W, the outer circumferential surface of the deformation part 191 is in close contact with the fixing member 193, and the fluid contained in the third space S3 may be prevented from leaking to the outside.

The pump 100 according to an embodiment of the present disclosure may be a small pump used in a device for injecting medicine such as insulin. However, the use of the pump is not particularly limited as long as the pump operates the shaft assembly 120 by using the structure and mechanism as described above.

The present disclosure has been described with reference to the embodiment illustrated in the drawings, but this is only an example. It will be understood by those of ordinary skill in the art that various modifications and variations may be made thereto. Accordingly, the true technical protection scope of the present disclosure should be defined by the technical spirit of the appended claims.

## Claims

1. An electroosmotic pump comprising:
a housing that provides an inner space for containing a fluid therein and
has a shaft hole connecting the inner space to an outer space thereof; and
a shaft assembly that covers the shaft hole from an outside of the housing, is connected to the housing, and is capable of changing a shape in a longitudinal direction by a flow of the fluid passing through the shaft hole.

2. The electroosmotic pump of claim 1, wherein the shaft assembly comprises:
an expansion/contraction member that forms an expansion/contraction space communicating with the shaft hole; and
a rod member that is coupled to the expansion/contraction member and is formed to extend while sharing a longitudinal central axis with the expansion/contraction member.

3. The electroosmotic pump of claim 2, wherein the expansion/contraction member comprises:
a body part that has an expansion/contraction space provided therein with an opening facing the shaft hole and is formed in a shape of a corrugated pipe;
a flange part extending radially from one end of the body part having the opening formed therein; and
a head part formed on another end opposite to the one end of the body part from which the flange part extends,
wherein the flange part is joined to the housing.

4. The electroosmotic pump of claim 2, wherein the expansion/contraction member includes a rubber material.

5. The electroosmotic pump of claim 1, further comprising a membrane that divides the inner space into a first space and a second space,
wherein the flow of the fluid passing through the shaft hole depends on a flow of the fluid passing through the membrane.
